(19) Europäisches Patentamt
European Patent Office
Office européen des brevets

(11) Veröffentlichungsnummer: **0 145 663**
**B1**

(12) **EUROPÄISCHE PATENTSCHRIFT**

(45) Veröffentlichungstag der Patentschrift: **21.06.89**

(51) Int. Cl.⁴: **C 07 D 249/08**, C 07 D 233/60, C 07 D 405/06, A 01 N 43/653, A 01 N 43/50

(21) Anmeldenummer: **84810589.6**

(22) Anmeldetag: **03.12.84**

(54) **1-Di- oder Triazo1-2,3-diphenyl-propan-2,3-diole als Herbizide und Wuchsregulatoren.**

(30) Priorität: **08.12.83 CH 6573/83**

(43) Veröffentlichungstag der Anmeldung: **19.06.85 Patentblatt 85/25**

(45) Bekanntmachung des Hinweises auf die Patenterteilung: **21.06.89 Patentblatt 89/25**

(84) Benannte Vertragsstaaten: **BE CH DE FR GB IT LI NL**

(56) Entgegenhaltungen:
**EP-A-0 070 798**
**EP-A-0 078 594**
**EP-A-0 066 173**

(73) Patentinhaber: **CIBA- GEIGY AG, Klybeckstrasse 141, CH- 4002 Basel (CH)**

(72) Erfinder: **Kunz, Walter, Dr., Buchenstrasse 9, CH- 4104 Oberwil (CH)**
Erfinder: **Rempfler, Hermann, Dr., Brücklismattstrasse 16, CH- 4107 Ettingen (CH)**
Erfinder: **Müller, Urs, Dr., Drosselstrasse 6, CH- 4142 Münchenstein (CH)**

**EP 0 145 663 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue 1-Di- oder Triazol-2,3-diphenyl-propan-2,3-diole und Derivate der untenstehenden Formel I sowie herbizide und den Pflanzenwuchs regulierende Mittel, welche diese Substanzen als Wirkstoffe enthalten. Die Erfindung betrifft ebenfalls die Herstellung der 1- Di- oder Triazolyl-2,3-diphenyl-propan-2,3-diole und der genannten Mittel sowie die Verwendung der Wirkstoffe oder Mittel zur Bekämpfung von Unkräutern und Regulierung des Pflanzenwuchses.

Die neuen 1-Di- oder Triazolyl-2,3-diphenyl-propan-2,3-diole und Derivate entsprechen der Formel I

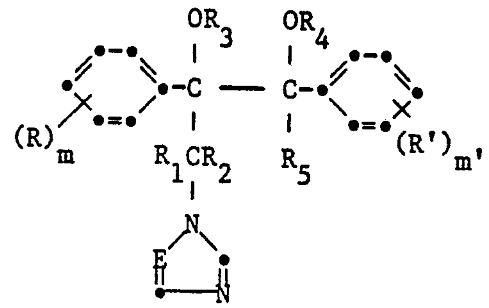

(I),

worin E ein Stickstoffatom oder die Methingruppe -CH=,

m und m' unabhängig voneinander 0, 1, 2 oder 3

R und R' unabhängig voneinander Halogen, $C_1$-$C_4$ Alkyl, $C_1$-$C_4$ Halogenalkyl, $C_1$-$C_4$ Alkoxy, $C_1$-$C_4$ Halogenalkoxy, Nitro, Cyano, $C_1$-$C_4$ Alkylthio, Phenyl oder Phenoxy, welches unsubstituiert oder gegebenenfalls durch $R_{(m)}$ substituiert ist, ferner $C_1$-$C_4$ Alkoxycarbonyl, $C_1$-$C_4$ Halogenalkylthio, Amino, Mono- oder Di($C_1$-$C_4$ alkyl)amino,

$R_1$ und $R_2$ unabhängig voneinander je Wasserstoff oder $C_1$-$C_4$ Alkyl,

$R_3$ und $R_4$ einzeln je Wasserstoff, $C_1$-$C_4$ Alkyl, $C_1$-$C_4$ Alkylcarbonyl,

$R_3$ und $R_4$ zusammen eine Methylenbrücke $-CR_6R_7-$,

$R_5$ Wasserstoff oder $C_1$-$C_4$ Alkyl,

$R_6$ und $R_7$ einzeln je Wasserstoff oder $C_1$-$C_4$ Alkyl,

$R_6$ und $R_7$ zusammen mit dem Kohlenstoffatom, an das sie gebunden sind einen 3 - 6 gliedrigen gesättigten oder ungesättigten Kohlenwasserstoffring bedeutet.

Unter dem Begriff Alkyl oder als Bestandteil eines anderen Substituenten sind je nach der Anzahl Kohlenstoffatome folgende Gruppen zu verstehen: Methyl, Äthyl, n-Propyl, Isopropyl, n-Butyl, Isobutyl, sek. und tert.Butyl.

Unter Halogen sollten Fluor, Chlor, Brom und Jod, vorzugsweise aber Chlor und Brom verstanden werden.

In Fällen wo das durch $R_3$ und $R_4$ gebildete Molekülfragment $-CR_6R_7$-ist, stellt diese einen 1,3-Dioxolanring dar. Der gesättigte oder ungesättigte Kohlenwasserstoffring, welcher von $-CR_6R_7$ gebildet werden kann ist beispielsweise Cyclopropyl, Cyclobutyl, Cyclopentyl, Cyclopentenyl, Cyclopentadienyl, Cyclohexyl, Cyclohexenyl oder Cyclohexadienyl.

Die Verbindungen der Formel I sind bei Raumtemperatur stabile Öle, Harze oder vor allem Feststoffe, welche sich durch wertvolle herbizide und den Pflanzenwuchs regulierende Eigenschaften auszeichnen. Sie lassen sich zur Bekänpfung unerwünschter Unkräuter z. B. in Nutzpflanzenkulturen sowie zur Regulierung des Pflanzenwuchses einsetzen. Dabei sind diejenigen 1- Di- oder Triazolyl-2,3-diphenylpropan-2,3-diole bevorzugt, welche

- der Formel Ia entsprechen

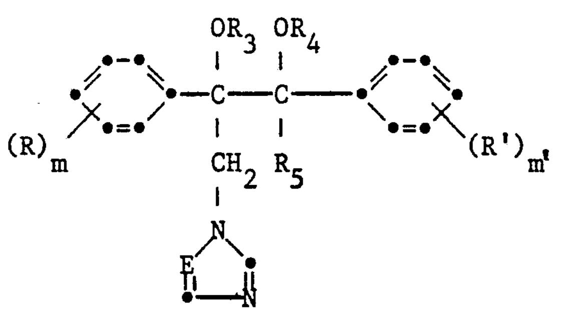

Ia

worin E, m, m', R, R', $R_3$, $R_4$ und $R_5$ die unter Formel I gegebene Bedeutung haben

- der Formel Ib entsprechen

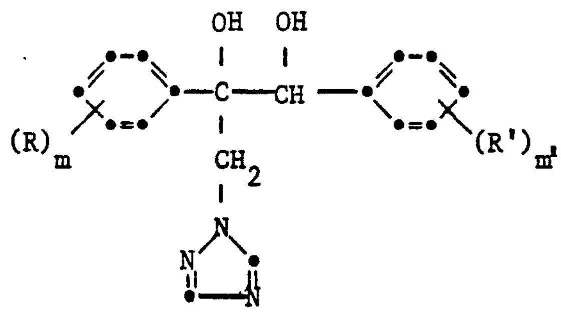

Ib

worin m und m' unabhängig voneinander 0,1 oder 2 und R und R' unabhänig voneinander Halogen, $C_1$-$C_4$ Alkyl, $C_1$-$C_4$ -Alkoxy oder $C_1$-$C_4$ Halogenalkyl bedeuten, besonders

2-(2,4-Dichlorphenyl)-3-phenyl-1-(1,2,4-triazol-1H-1-yl)-propan-2,3-diol, 2-(4-Chlorphenyl)-3-phenyl-1-(1,2,4-triazol-1H-1-yl)-propan-2,3-diol, 2-(4-Chlorphenyl)-3-(2-chlorphenyl)-1-(1,2,4-triazol-1H-1-yl)-propan-2,3-Diol, 2-(4-Chlorphenyl)-3-para-tolyl-1-(1,2,4-triazol-1H-1-yl)-propan-2,3-diol 2-(4-Chlorphenyl)-3-(4-methoxyphenyl)-1-(1,2,4-triazol-1H-1-yl)-propan-2,3-diol und
2-(4-Chlorphenyl)-3-(4-fluorphenyl)-1-(1,3,4-triazol-1H-1-yl)-propan-2,3-diol;
- der Formel Ic entsprechen

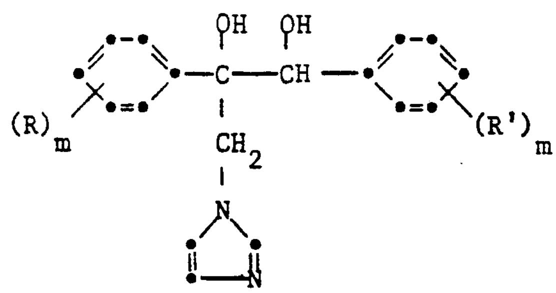

Ic,

worin m und m' unabhängig voneinander 0, 1 oder 2 und R und R' unabhängig voneinander Halogen, $C_1$-$C_4$ Alkyl, $C_1$-$C_4$ Alkoxy oder $C_1$-$C_4$ Halogenalkyl bedeuten:
- der Formel Id entsprechen

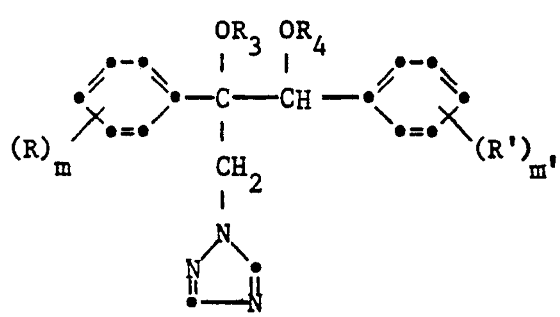

Id

worin m und m' unabhängig voneinander 0, 1 oder 2 und
R und R' unabhängig voneinander Halogen, $C_1$-$C_4$ Alkyl, $C_1$-$C_4$ Alkoxy oder $C_1$-$C_4$ Halogenalkyl
$R_3$ und $R_4$ je einzeln $C_1$-$C_4$ Alkyl, $C_1$-$C_4$ Alkylcarbonyl oder eines auch Wasserstoff oder zusammen eine 3 - 6 gliedrige Alkylenbrücke, die ein- oder zweifach durch $C_1$-$C_4$ Alkyl substituiert sein kann, besonders 2,2-Dimethyl-4-phenyl-5-(2,4-dichlorphenyl)-3-(1,2,4-triazol-1H-1-yl-methyl)-1,3-dioxolan oder
1-(1,3,4-Triazol-1H-1-yl)-2-(2,4-dichlorphenyl)-2-methoxy-3-phenyl-propan-3-ol.

Die Verbindungen der Formel I werden durch verschiedene z. T. bekannte Synthesewege hergestellt, wie beispielsweise in der schweiz. Patentanmeldung Nr. 6822/83-2 beschrieben.

Die Verbindungen der Formel I werden z. B. hergestellt, indem man ein α-Haloketon der Formel II

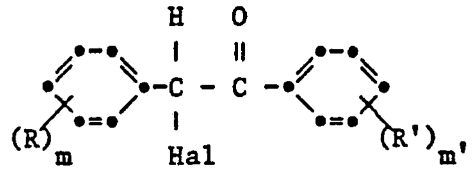

(II)

mit Paraformaldehyd oder einem Keton $O=CR_1R_2$ und einer nicht nukleophilen Base, wie z. B. einem Alkalihydrid (LiH, NaH, KH, K-tert. Butylat oder $NaOC_2H_5$) reagieren lässt und anschliessend Imidazol oder 1,2,4-Triazol als solches oder in Form eines Alkalimetallsalzes zugibt, wobei ein Alkohol der Formel III entsteht,

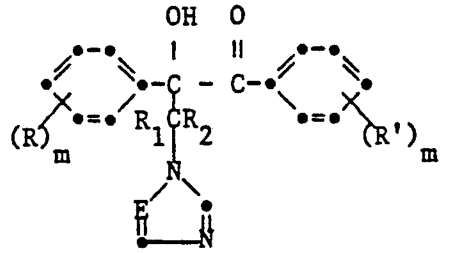

(III),

welcher durch Behandeln mit einem komplexen Hydrid wie Natriumborhydrid, Lithiumaluminiumhydrid oder einer Lithiumalkylverbindung $LiR_5$ oder einem Magnesiumhalogenid $R_5MgHal$ zum Diol der Formel IV, worin $R_3$ und $R_4$ Wasserstoff bedeuten, umgesetzt wird.

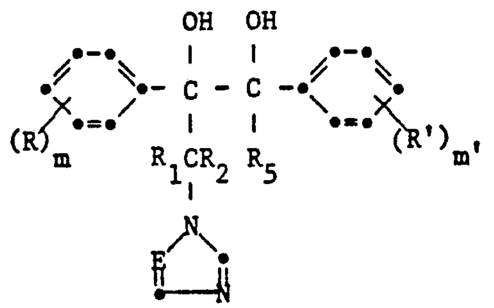

(IV)

Wenn $R_5$ Wasserstoff sein soll, kann man die Reduktion auch mit Wasserstoff und einem Hydrierungskatalysator vornehmen.

Die Verbindungen der Formel III können auch hergestellt werden, indem man eine Desoxybenzoin-Verbindung der Formel VI mit einem Aldehyd oder Keton der Formel VI kondensiert

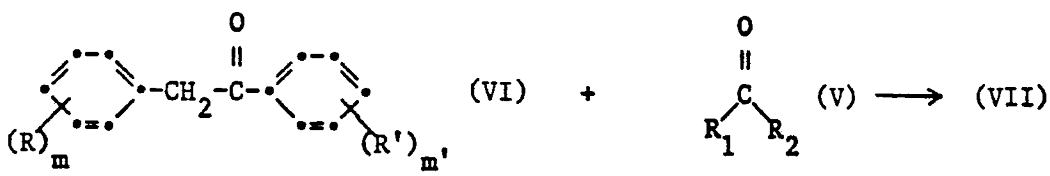

(VI) + (V) (VII)

und die entstandene Verbindung der Formel VII

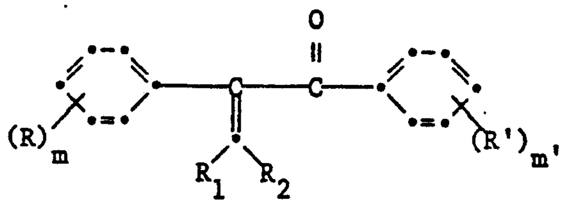

(VII)

anschliessend mit einer Persäure oder mit Wasserstoffperoxid in Gegenwart einer Base wie z. B. Natronlauge in das entsprechende Epoxid der Formel VIII überführt,

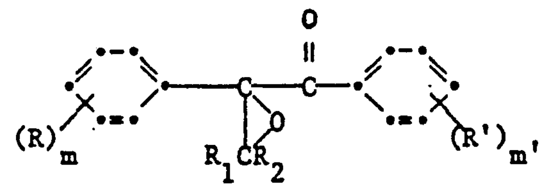

(VIII),

welches dann durch Kondensation mit einem Alkalimetallsalz eines Imidazols oder 1,3,4-Triazols, in einem inerten organischen Lösungsmittel zu einer Verbindung der Formel III umgesetzt wird.

In den obigen Formeln haben E m, m' R, R', R , $R_2$ und $R_5$ die unter Formel I gegebene Bedeutung. Hal steht für ein Halogenatom, vorzugsweise Chlor oder Brom.

Eine Verbindung der Formel IV lässt sich auch durch Reaktion mit je einem Mol einer Verbindung der Formel

$R_3$-X und $R_4$-X

oder mit 2 Mol einer dieser Verbindungen, worin X für eine übliche Abgangsgruppe, wie z. B. ein Halogenatom, vorzugsweise Chlor oder Brom, eine Sulfonyloxygruppe, ein Isoharnstoffrest oder eien Acyloxygruppe steht, in einem inerten organischen Lösungsmittel in Gegenwart einer Base als säurebindendes Mittel, in einer oder zwei Stufen zum Äther der Formel I umwandeln.

Ein Rest $R_3$ kann z. B. als Halogenid auch direkt mit dem Hydroxylrest der Formel III zum Äther verbunden werden, entsprechend dem Reaktionsschema

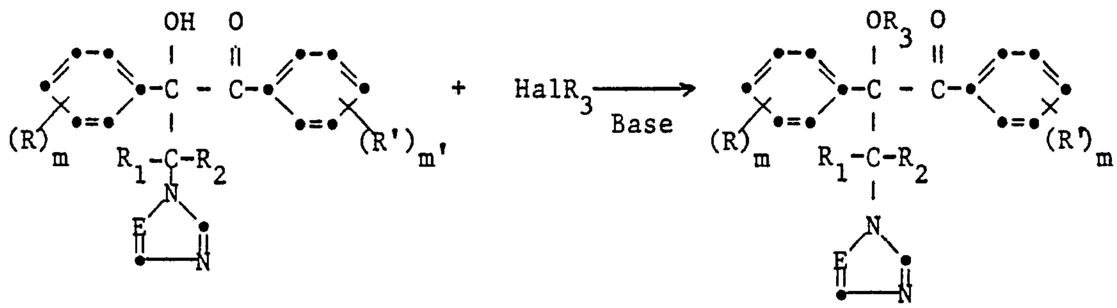


(III) (IIIa)

Die Verbindung der Formel IIIa wird dann durch Behandeln mit einem komplexen Metallhydrid, einer Lithiumalkylverbindung $LiR_5$ oder einem Magnesiumhalogenid $R_5Mg$ Hal weiterbehandelt zu einer Verbindung der Formel I, in der $R_4$ Wasserstoff bedeutet, welche man anschliessend in einem inerten organischen Lösungsmittel, in Gegenwart einer Base mit einem Mol einer Verbindung der Formel $R_4$-X reagieren kann, um zu einer Äther-Verbindung der Formel I zu kommen.

Eine Verbindung der Formel Ic kann in Gegenwart einer starken Säure mit einem Aldehyd oder Keton der Formel

$O = C\text{-}R_6$ oder dessen Ketal
|
$R_7$

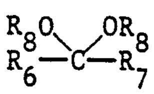 zu einem Glykoläther der Formel Ie kondensiert werden

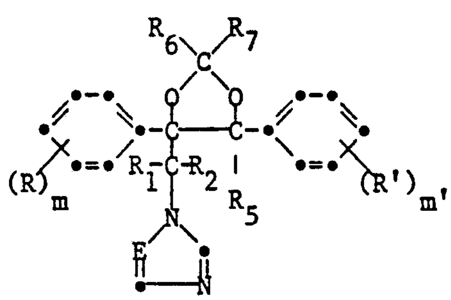

(Ie),

worin E, m, m', R, R', $R_1$, $R_2$, $R_5$, $R_6$ und $R_7$ die unter Formel I gegebene Bedeutung haben und $R_8$ $C_{1\text{-}6}$ Alkyl bedeutet.

Das erfindungsgemässe Verfahren ist dadurch gekennzeichnet, dass man eine Verbindung der Formel III

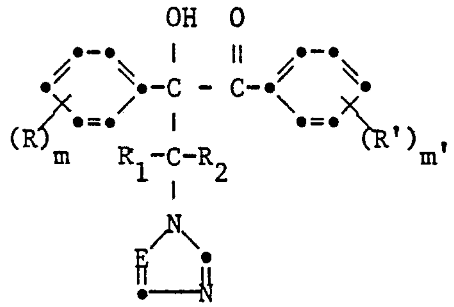

(III),

worin E, m, m', R, R', R und R die unter Formel I gegebene Bedeutung haben in einem inerten organischen Lösungsmittel reduziert, mit einem komplexen Metallhydrid, mit Wasserstoff und einem Katalysator, einer Lithium(alkyl)verbindung $LiR_5$ oder einem Magnesiumhalogenid $R_5Mg$ Hal, und das erhaltene Diol der Formel Ic wie vorstehend beschrieben umsetzt.

Verbindungen der Formel Id, in denen $R_1$, $R_2$ und $R_3$ Wasserstoff und $R_4$ $C_1$-$C_4$ Alkyl bedeuten können auch hergestellt werden, indem man ein substituiertes Benzoin der Formel IX

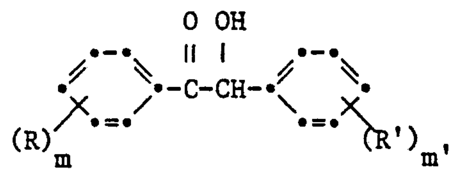

(IX),

worin m, m', R und R' die unter Formel I gegebene Bedeutung haben, mit einer Verbindung der Formel $R'_4$-X, worin $R'_4$ eine $C_1$-$C_4$ Alkylgruppe und X eine Abgangsgruppe, ein Halogenatom oder ein Sulfonyloxy-, Isoharnstoff- oder Acyloxyrest bedeutet, in einem inerten organischen Lösungsmittel, in Gegenwart der äquimolaren Menge einer Base wie z. B. Natriumhydrid, alkyliert, anschliessend die Ketogruppe mittels Dimethylsulfoxoniummethylid epoxidiert zum Epoxid der Formel X

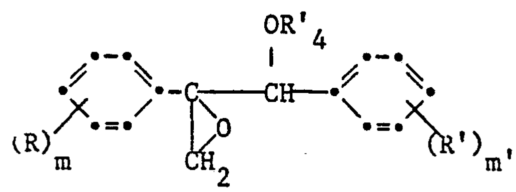

(X),

worin m, m', R, R' und die unter Formel I gegebene Bedeutung haben und $R'_4$ eine $C_1$-$C_4$ Alkylgruppe darstellt, und anschliessender Öffnung des Epoxidringes mit Imidazol oder 1,2,4-Triazol in Gegenwart einer Base.

Eine weitere Methode zur Herstellung von Di- oder Triazolyl-2,3-diphenylpropan-2,3-diolen der Formel Ib besteht darin, dass man das Benzoin der Formel IX mittels Formaldehyd in Gegenwart einer Base hydroxymethyliert, gemäss dem Schema:

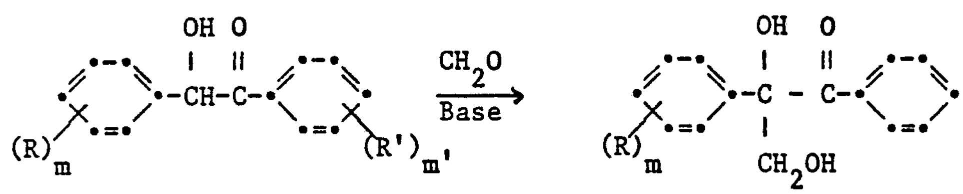


(IX) (XI) ,

und die entstandene 2,3-Diphenyl-1,2-hydroxy-3-oxopropan-Verbindung der Formel XI, am primären Alkoholrest zu einem reaktionsfähigen Ester wie. z. B. mit Methansulfonylchlorid verestert dann mit Imidazol oder 1,2,4-Triazol in Gegenwart einer Base umsetzt und schliesslich die Ketogruppe der erhaltenen Verbindung der Formel XII

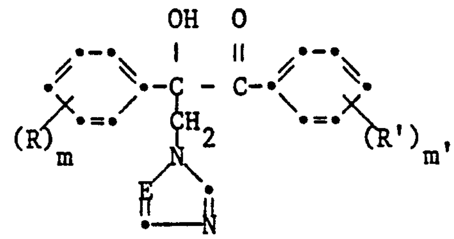

(XII)

reduziert, z. B. mit einem komplexen Hydrid oder mit Wasserstoff in Gegenwart eines Katalysators.

In den obigen Formeln haben E, m, m', R und R' die unter Formel I gegebene Bedeutung.

Die Reaktionen werden gegebenenfalls in Gegenwart von Basen als Säureakzeptoren oder Kondensationsmitteln durchgeführt. Als solche kommen organische und anorganische Basen in Betracht wie z. B. sekundäre oder tertiäre Amine (Diäthylamin, Triäthylamin, Di- und Tripropylamin), Pyridin und Pyridinbasen, (4-Dimethylaminopyridin, 4-Pyrrolidylaminopyridin), Oxide, Hydride, Hydroxide, Carbonate und Hydrogencarbonate von Alkali und Erdalkalimetallen. Darüber hinaus kommen auch Alkalimetallacetate in Frage, Alkalimetallalkoholate.

Es kann auch von Vorteil sein, das 1,2,4-Triazol oder Imidazol als Alkalimetallsalz einzusetzen, indem man es vorerst z. B. in situ mit einem Alkalimetallalkoholat umsetzt.

Als inerte organische Lösungsmittel werden bevorzugt N,N-Dimethylformamid, N,N-Dimethylacetamid, Dimethylsulfoxid, Acetonitril, Benzonitril verwendet. Diese werden allein oder in Kombination mit anderen reaktionsinerten Lösungsmitteln wie Benzol, Toluol, Xylol, Hexan, Petroläther, Chlorbenzol oder Nitrobenzol verwendet.

Die Kondensationen finden im Temperaturbereich von -10° bis zum Siedepunkt des Lösungsmittels statt.

Die Umsetzung des α-Haloketons der Formel II mit Paraformaldehyd oder einem Keton

$O=C-R_1$ findet in Gegenwart
|
$R_2$

einer starken, nicht nukleophilen Basen, wie einem Alkalimetallhydrid (NaH) und anschliessender Zugabe des Imidazols oder 1,2,4-Triazols oder bevorzugt eines Alkalimetallsalzes desselben statt. Die Reaktion erfolgt in einem Temperaturbereich von 0°- ca. 140° C, vorzugsweise 10 - 80° und wird in wasserfreien Lösungsmitteln, wie z. B. N,N-Dimethylformamid, N,N-Dimethylacetamid, Dimethylsulfoxid, Hexamethylphosphortriamid, N-Methylpyrrolidon, Acetonitril, Äther, Tetrahydrofuran oder Dioxan durchgeführt.

Die Ausgangsstoffe der Formel II sind bekannt oder lassen sich in bekannter Art und Weise, z. B. durch Chlorierung von Benzoinen der Formel IX herstellen.

Die Desoxybenzoine der Formel VI sind bekannt oder können durch Friedel Crafts Reaktion aus einem Phenylessigsäurehalid und einem Benzol hergestellt werden gemäss dem Schema:

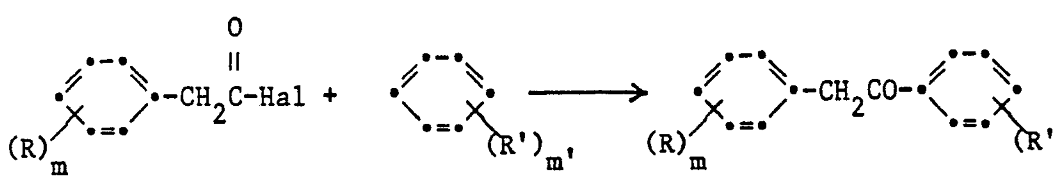

(VI)

Die Verbindungen der Formel VI können ebenfalls erhalten werden durch Kondensation eines Benzoesäureesters mit einem Benzylnitril, anschliessender Hydrolyse der Cyanogruppe und Decarboxylierung des dadruch entstandenen Carboxylesters gemäss dem Schema:

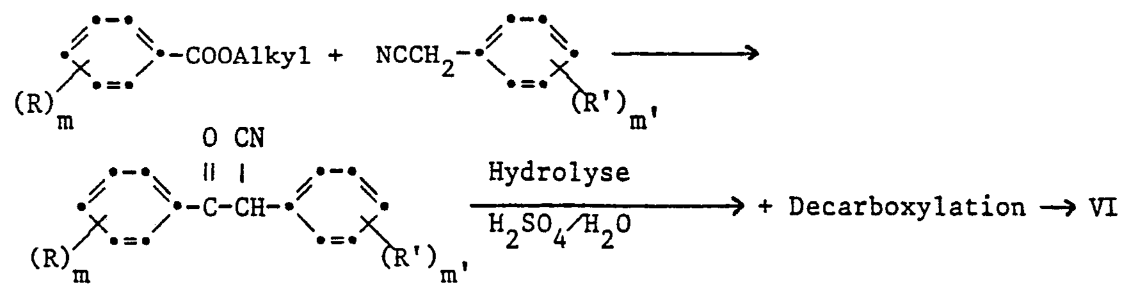


Die Verbindungen der Formel VI können beispielsweise auch durch eine Grignard-Reaktion aus einem Benzoesäuresalz vorzugsweise dem Lithiumsalz und einem Benzylmagnesiumhalogenid hergestellt werden, gemäss dem Schema:

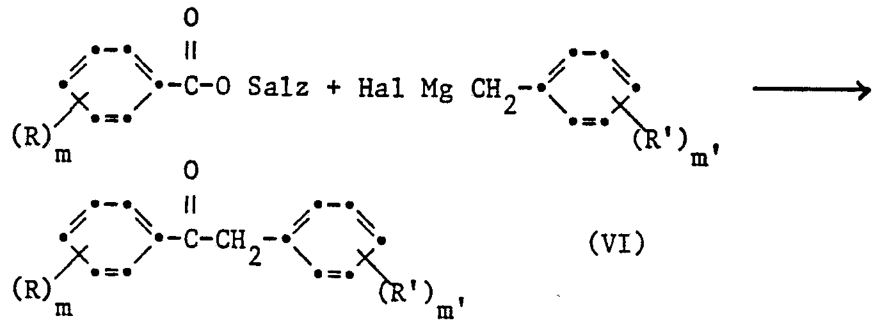

(VI)

Hydrierungen und Reduktionen werden in wasserfreien inerten Lösungsmitteln, wie Äther, Dioxan, Tetrahydrofuran, Benzol, Toluol, Xylol etc. vorgenommen. Als Reduktionsmittel können die komplexen Metallhydride wie Lithiumaluminiumhydrid, Natriumborhydrid, Alkalimetallhydride oder auch Wasserstoff mit Katalysatoren wie z. B. Palladium oder Platin auf Aktivkohle verwendet werden. Falls ein Alkylrest $R_5$ einzuführen ist empfiehlt es sich ein $R_5$-Magnesiumhalogenid oder eine Lithium, Kalium oder Natriumverbindung von $R_5$ zu verwenden.

Die 1-Di- und Triazolyl-2,3-diphenyl-propan-2,3-diole der Formel I besitzen in der 2- und 3-Stellung asymmetrisch Kohlenstoffatome und können in enantiomeren und diastereomeren Formen vorliegen. Diese lassen sich auf übliche Weise, z. B. durch fraktionierte Kristallisation von Salzen mit optisch aktiven starken Säuren oder Basen in die reinen optischen Antipoden aufspalten. Diastereomere lassen sich voneinander beispielsweise durch Säulenchromatographie trennen und erst dann in die Enantiomeren aufspalten.

Die Enantiomeren können unterschiedliche biologische Wirkungen aufweisen, wobei bei gleichem Wirkungsspektrum ein gradueller Unterschied in der Wirkung vorliegen kann. Die vorliegende Erfindung betrifft alle reinen Stereoisomeren, Enantiomeren und die Gemische untereinander in welcher die Verbindungen der Formel I vorliegen können.

Die beschriebenen Herstellungsverfahren sind, einschliesslich aller Teischritte, ein wichtiger Bestandteil der vorliegenlen Erfindung.

Es wurde nun überraschend gefunden, dass die neuen Wirkstoffe der Formel I bzw. Mittel, die diese Wirkstoffe enthalten sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in

Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z. B. mehr oder grössere Früchte zur Ausbildung kommen.

Ertragssteigerungen können in manchen Fällen auch durch einen Eingriff in den pflanzlichen Stoffwechsel, wie z. B. durch Erhöhung der Photosyntheseleistung, erreicht werden, ohne dass sich Änderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z. B. Interesse bei der Stecklingsvermehrung von Pflanzen. Es ist jedoch auch möglich, das Wachstum der Seitentriebe zu hemmen, z. B. um bei Tabakpflanzen nach der Dekapitierung die Ausbildung von Seitentrieben zu verhindern und damit das Blattwachstum zu fördern.

Durch Einsatz von Wachstumsregulatoren lässt sich der vorzeitige Fruchtfall verhindern. Es ist jedoch auch möglich, den Fruchtfall, - zum Beispiel bei Obst - im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z. B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samen- oder Knospenruhe der Pflanzen also die endogene Jahresrhythmik, beeinflusst werden, so dass die Pflanzen, wie z. B. Ananas oder Zierpflanzen in Gärtnereien zu einem Zeitpunkt keimen, austreiben oder blühen an dem sie normalerweise hierzu keine Bereitschaft zeigen.

Mit Wachstumsregulatoren kann auch erreicht werden, dass der Austrieb von Knospen oder die Keimung von Samen verzögert wird, z. B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbeckungspflanzen, den sogenannten Cover crops. In tropischen und subtropischen Monokulturen, wie z. B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen.

Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Äpfel, Birnen Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer): oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Die Verbindungen der Formel I besitzen ebenfalls herbizide Wirkung. In grösseren Aufwandmengen können sie als Totalherbizide zur Freihaltung von Verkehrswegen, Plätzen, Sportanlagen, Kanalisationen etc. von Pflanzenwuchs verwendet werden. In geringerer Aufwandmenge eignen sie sich zur selektiven Bekämpfung von Unkraut in grossflächigen Kulturen von Nutzpflanzen wie z. B. Getreide, Mais, Hirse, Reis, Soja, Baumwolle, Raps, Sonnenblumen, Mohn etc.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiterem in der Formulierumgstechnik üblichen Trägerstoffen, Tensidem oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige

Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$ - $C_{22}$), wie z. B. die Na- oder K-Salze der Öl- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate, wie z. B. Salze des Phosphorsäureesters eines p-Nonylphenol-(4-14)-Äthylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxyaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)äthylammoniumbromid.

Die in Der Formulierungstechnik gebräuchlichen Tenside sind u. a. in folgenden Publikationen beschrieben:

"MC Cutcheon's Detergents and Emulsifiers Annual" MC
Publishing Corp., Ringwood, New Jersey, 1979.
J. and M. Ash, "Encyclopedia of Surfactants" Col. I - III,
Chemical Publishing Co., Inc. New. York, 1980 - 81.
H. Stache "Tensid Taschenbuch" 2. Auflage
C. Hanser Verlag, München und Wien, 1981.

Diese Zubereitungen enthalten in der Regel 0,1 bis 99 % insbesondere 0,1 bis 95 %, Wirkstoffe der Formel I, 1 bis 99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel, sowie Dünger oder andere Wirkstoffe zur Erzielung spezieller Effekte enthalten.

In den nachfolgenden Beispielen sind die Temperaturen in Celsiusgraden angegeben, Prozente und Angaben von "Teilen" beziehen sich auf das Gewicht.

**Beispiel 1: Verfahren zur Herstellung von 2-(2,4-Dichlorphenyl)-1-phenyl-3-(1,2,4-triazol-1-yl)-propan-1,2-diol**

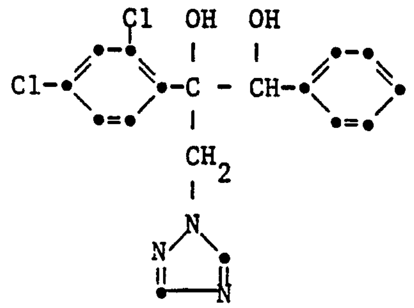

Man löst 45 g 1-Benzoyl-1-(2,4-dichlorphenyl)-2-(1H-1,2,4-triazol-1-yl)ethanol in 300 ml Methanol und gibt dazu unter Rühren und Kühlen auf 5 - 10° portionenweise 7,4 g Natriumborhydrid ($NaBH_4$). Dann wird während 15 Stunden bei Raumtemperatur nachgerührt, worauf man unter Stickstoffatmosphäre tropfenweise 50 ml konzentrierte Salzsäure zugibt.

Das Reaktionsgemisch wird abermals 5 Stunden bei Raumtemperatur gerührt und dann eingedampft. Der Rückstand wird dreimal in ca. 100 ml Methanol aufgenommen und wieder eingedampft. Anschliessend stellt man den Rückstand mit 10 %-iger wässriger Natronlauge stark alkalisch (pH 12) und extrahiert mit Methylenchlorid. Die organische Phase wird mit Wasse gewaschen, über Natriumsulfat getrocknet und eingedampft. Der Rückstand wird aus Tetrahydrofuran/Hexan umkristallisiert und man erhält das Titelprodukt als weisse Kristalle mit Schmelzpunkt 187 - 189°.

In analoger Weise zu diesem Beispiel werden folgende Verbindungen hergestellt:

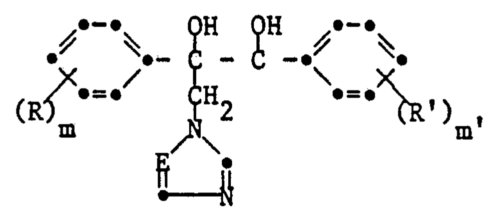

**Tabelle 1:**

| No. | E | $(R)_m$ | $(R')_{m'}$ | |
|---|---|---|---|---|
| 1.01 | N | 2,4-$Cl_2$ | - | Smp. 187 - 189° |
| 1.02 | N | - | - | |
| 1.03 | N | 4-CL | - | Smp. 147 - 148° |
| 1.04 | N | 4-Cl | 4-Cl | Smp. 168 - 170° |
| 1.05 | N | 4-Cl | 2-Cl | Smp. 180 -181° |
| 1.06 | N | 4-Cl | 2,4-$Cl_2$ | |
| 1.07 | N | 4-F | 4-Cl | Smp. 153 - 154° |
| 1.08 | N | 4-$CF_3$ | - | |
| 1.09 | N | 4-$CF_3$ | 4-Cl | Smp. 148 - 150° |
| 1.10 | N | 4-$CF_3$ | 2,6-$Cl_2$ | |
| 1.11 | N | 4-$CF_3$ | 2,4-$Cl_2$ | |
| 1.12 | N | 4-$CF_3$ | 4-$CH_3$ | |
| 1.13 | N | 4-Br | 4-Cl | Smp. 178 - 180° |
| 1.14 | N | 4-Br | - | Smp. 140 - 141° |
| 1.15 | N | 4-Br | 2-Cl | |
| 1.16 | N | 4-Br | 2,4-$Cl_2$ | |
| 1.17 | N | 2-Br | 4-$CH_3$ | |
| 1.18 | N | 3-$CF_3$ | - | |
| 1.19 | N | 3-$CF_3$ | 4-Cl | Smp. 148 - 150° |
| 1.20 | N | 3-$CF_3$ | 3-$CF_3$ | |
| 1.21 | N | 3-$CF_3$ | 4-$CH_3$ | |
| 1.22 | N | 3-$CF_3$ | 2,3-$Cl_2$ | |
| 1.23 | N | 2-F | - | |

| | | | | |
|---|---|---|---|---|
| 1.24 | N | 2. Cl | - | Smp. 176 - 178° |
| 1.25 | N | 2-Cl | 4-Cl | Smp. 172 - 174° |
| 1.26 | N | 4-$OC_6H_4Cl$ | - | |
| 1.27 | N | 2,4-$Cl_2$ | 2,4-$(CH_3)_2$ | Diastereomer A Smp. 192 - 196° Diastereomer B Smp. 188 - 191° |
| 1.28 | N | 4-F | 4-F | |
| 1.29 | N | 3-F | 4-F | |
| 1.30 | N | 4-Cl | 4-Br | Smp. 60 - 62° |
| 1.31 | N | 4-Cl | 3-$CF_3$ | |
| 1.32 | N | 4-Cl | 4-F | Smp. 163 - 164° |
| 1.33 | N | 4-Cl | 4-$CH_3$ | Smp. 169 - 170° |
| 1.34 | N | 4-Cl | 4-$OCH_3$ | Smp. 147 - 149° |
| 1.35 | N | 4-Cl | 4-$CF_3$ | Smp. 185 - 187° |
| 1.36 | N | 4-Cl | 3,4 $Cl_2$ | Smp. 170 - 172° |
| 1.37 | N | 4-Cl | 2-F | Smp. 164 - 166° |
| 1.38 | N | 4-Cl | 2-$CH_3$ | Smp. 136 - 138° |
| 1.39 | N | 4-Cl | 3-Cl | Smp. 149 - 150° |
| 1.40 | N | 4-Cl | 2,5-$Cl_2$ | |
| 1.41 | N | 4-F | - | Smp. 188 - 189° |
| 1.42 | N | 4-Cl | 2-$OCH_3$ 2,6-$Cl_2$ | |
| 1.43 | N | 4-$CH_3$ | | Smp. 135 - 136 |
| 1.44 | N | 4-$CH_3$ | 2-Cl | |
| 1.45 | N | 4-$CH_3$ | 4-Cl | |
| 1.46 | N | - | 2-Cl | |
| 1.47 | N | - | 3-Cl | Smp. 148 - 150° |
| 1.48 | N | - | 4-Cl | Smp. 145 - 146° |
| 1.49 | N | - | 4-$OCH_3$ | |
| 1.50 | N | 3-Cl | - | Smp. 135 - 137° |
| 1.51 | N | 3-Cl | 4-Cl | Smp. 123 - 125° |
| 1.52 | N | - | 2,4-$Cl_2$ | |
| 1.53 | N | - | 4-F | |
| 1.54 | N | - | 2-F | |
| 1.55 | N | 2,4-$Cl_2$ | 2,4-$Cl_2$ | Smp. 109 - 113° |
| 1.56 | N | 2,4-$Cl_2$ | 2-Cl, 4-$CH_3$ | |
| 1.57 | N | 2,4-$Cl_2$ | 4-$CH_3$ | Smp. 176 - 180° |
| 1.58 | N | 2,4-$Cl_2$ | 4-Cl | Smp. 178 - 190° |
| 1.59 | N | 2,4-$Cl_2$ | 4-$OCH_3$ | |
| 1.60 | N | 2,4-$Cl_2$ | 2-Cl | |
| 1.61 | N | 2,4-$Cl_2$ | 4-F | |
| 1.62 | N | 2,4-$Cl_2$ | 2-F | |
| 1.63 | CH | 2,4-$Cl_2$ | - | |
| 1.64 | CH | - | - | |
| 1.65 | CH | 4-Cl | - | |
| 1.66 | CH | 4-Cl | 4-Cl | |
| 1.67 | CH | 4-Cl | 2-Cl | |
| 1.68 | CH | 4-Cl | 2,4-$Cl_2$ | |
| 1.69 | CH | 4-F | 4-Cl | |
| 1.70 | CH | 4-$CF_3$ | - | |
| 1.71 | CH | 4-$CF_3$ | 4-Cl | |
| 1.72 | CH | 4-$CF_3$ | 2,6-$Cl_2$ | |
| 1.73 | CH | 4-$CF_3$ | 2,4-$Cl_2$ | |
| 1.74 | CH | 4-$CF_3$ | 4-$CH_3$ | |
| 1.75 | CH | 4-Br | 4-Cl | |
| 1.76 | CH | 4-Br | - | |
| 1.77 | CH | 4-Br | 2-Cl | |
| 1.78 | CH | 4-Br | 2,4-$Cl_2$ | |
| 1.79 | CH | 2-Br | 4-$CH_3$ | |
| 1.80 | CH | 3-$CF_3$ | - | |
| 1.81 | CH | 3-$CF_3$ | 4-Cl | |
| 1.82 | CH | 3-$CF_3$ | 3-$CF_3$ | |
| 1.83 | CH | 3-$CF_3$ | 4-$CH_3$ | |
| 1.84 | CH | 3-$CF_3$ | 2,3-$Cl_2$ | |
| 1.85 | CH | 2-F | - | |

| | | | |
|---|---|---|---|
| 1.86 | CH | 2-Cl | - |
| 1.87 | CH | 2-Cl | 4-Cl |
| 1.88 | CH | 4-$OC_6H_4Cl$ | - |
| 1.89 | CH | 2,4-$Cl_2$ | 2,4 $(CH_3)_2$ |
| 1.90 | CH | 4-F | 4-F |
| 1.91 | CH | 3-F | 4-F |
| 1.92 | CH | 4-Cl | 4-Br |
| 1.93 | CH | 4-Cl | 3-$CF_3$ |

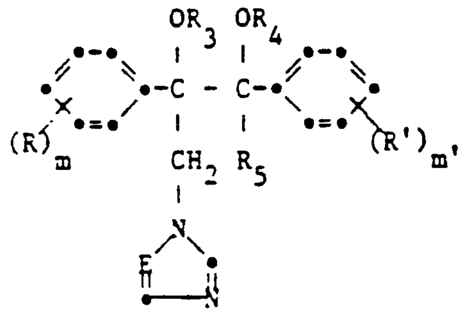

**Tabelle 2:**

| No. | E | $(R)_m$ | $R_3$ | $R_4$ | $R_5$ | $(R')_{m'}$ | |
|---|---|---|---|---|---|---|---|
| 2.01 | N | 2,4 $Cl_2$ | $CH_3$ | H | $CH_3$ | - | |
| 2.02 | N | 2,4 $Cl_2$ | -$C(CH_3)_2$- | | H | - | Smp. 162 - 165° |
| 2.03 | N | 2,4 $Cl_2$ | -$C(C_2H_5)_2$- | | H | 2,4 $Cl_2$ | |
| 2.04 | N | 4-Cl | $CH_3$ | H | $C_3H_7n$ | - | |
| 2.05 | N | 4-Cl | $CH_3$ | H | $C_3H_7i$ | - | |
| 2.06 | N | 4-Cl | $CH_3$ | H | $CH_3$ | - | |
| 2.07 | N | 4-Cl | $CH_2CH=CH_2$ | H | H | - | |
| 2.08 | N | 4-Cl | -$C(CH_3)_2$- | | H | 4-Cl | |
| 2.09 | N | 4-Cl | -$C(CH_3)_2$- | | H | 4-Br | |
| 2.10 | N | 4-Cl | -$C(CH_3)_2$- | | H | 3-$CF_3$ | |
| 2.11 | N | 4-Cl | -$C(CH_3)_2$- | | H | 4-$CH_3$ | |
| 2.12 | N | 4-Cl | -$C(CH_3)_2$- | | H | 4-F | |
| 2.13 | N | 4-F | -$C(CH_3)_2$- | | H | 4-F | |
| 2.14 | N | 3-F | -$C(CH_3)_2$- | | H | 3-F | |
| 2.15 | CH | 2,4 $Cl_2$ | $CH_3$ | H | $CH_3$ | - | |
| 2.16 | CH | 2,4 $Cl_2$ | -$C(CH_3)_2$- | | H | - | |
| 2.17 | CH | 2,4 $Cl_2$ | -$C(C_2H_5)_2$- | | H | 2,4 $Cl_2$ | |
| 2.18 | CH | 4-Cl | $CH_3$ | H | $C_3H_7n$ | - | |
| 2.19 | CH | 4-Cl | $CH_3$ | H | $C_3H_7i$ | - | |
| 2.20 | CH | 4-Cl | $CH_3$ | H | $CH_3$ | - | |
| 2.21 | CH | 4-Cl | $CH_2CH=CH_2$ | H | H | - | |
| 2.22 | CH | 4-Cl | -$C(CH_3)_2$- | - | H | 4-Cl | |
| 2.23 | CH | 4-Cl | -$C(CH_3)_2$- | | H | 4-Br | |
| 2.24 | CH | 4-Cl | -$C(CH_3)_2$- | | H | 3-$CF_3$ | |
| 2.25 | CH | 4-Cl | -$C(CH_3)_2$- | | H | 4-$CH_3$ | |
| 2.26 | CH | 4-Cl | -$C(CH_3)_2$- | | H | 4-F | |
| 2.27 | CH | 4-F | -$C(CH_3)_2$- | | H | 4-F | |
| 2.28 | CH | 3-F | -$C(CH_3)_2$- | | H | 3-F | |
| 2.29 | CH | 2,4-$Cl_2$ | $CH_3$ | H | $CH_3$ | - | |
| 2.30 | N | 2,4-$Cl_2$ | -$C(CH_3)_2$- | | H | 2,4-$Cl_2$ | |
| 2.31 | N | 2,4-$Cl_2$ | -$C(CH_3)_2$- | | H | 4-Cl | Smp. 183 - 185° |
| 2.32 | N | 2,4-$Cl_2$ | -$C(CH_3)_2$- | | H | 4-$Cl_3$ | Smp. 55 - 57° |
| 2.33 | N | 2,4-$Cl_2$ | $CH_3$ | $CH_3$ | H | 4-Cl | Smp. 65 - 102° |
| 2.34 | N | 2,4-$Cl_2$ | $C_2H_5$ | $C_2H_5$ | H | 2,4-$Cl_2$ | |
| 2.35 | N | 2,4-$Cl_2$ | $CH_3$ | H | H | 2,4-$Cl_2$ | |
| 2.36 | N | 2,4-$Cl_2$ | $C_2H_5$ | H | H | 2,4-$Cl_2$ | Smp. 59 - 68° |

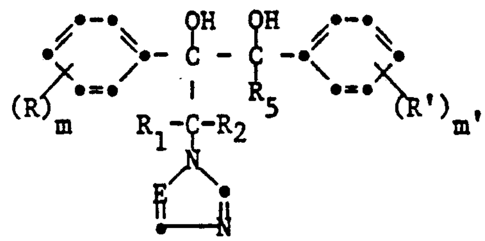

**Tabelle 3:**

| No. | E | $(R)_m$ | $R_1$ | $R_2$ | $R_5$ | $(R')_{m'}$ |
|---|---|---|---|---|---|---|
| 3.01 | N | 2,4 $Cl_2$ | $C_2H_5$ | H | H | - |
| 3.02 | N | 2,4 $Cl_2$ | $C_3H_7n$ | H | H | - |
| 3.03 | N | 4-Cl | $C_2H_5$ | H | H | - |
| 3.04 | N | 4-Cl | $CH_3$ | H | H | 4-Cl |
| 3.05 | N | 4-Cl | $C_2H_5$ | H | H | - |
| 3.06 | CH | 2,4 $Cl_2$ | $C_2H_5$ | H | H | - |
| 3.07 | CH | 2,4 $Cl_2$ | $C_3H_7n$ | H | H | - |
| 3.08 | CH | 4-Cl | $C_2H_5$ | H | H | - |
| 3.09 | CH | 4-Cl | $CH_3$ | H | H | 4-Cl |
| 3.10 | CH | 4-Cl | $C_2H_5$ | H | H | 4-Cl |

Formulierungsbeispiele

Die Verbindungen der Formel I werden im allgemeinen nicht als solche in der Landwirtschaft eingesetzt. Man verwendet gebrauchsfertig formulierte Mittel, welche entweder direkt oder mit Wasser verdünnt eingesetzt werden können.

**Beispiel 2: Stäubemittel**

Zur Herstellung eines a) 5 %-igen und b) 2 %-igen Stäubemittels werden die folgenden Stoffe verwendet:

a) 5 Teile Wirkstoff der Formel I
95 Teile Talkum,
b) 2 Teile des obigen Wirkstoffes oder einer Mischung,
1 Teil hochdisperse Kieselsäure,
97 Teile Talkum.

Die Wirkstoffe werden mit den Trägerstoffen vermischt und vermahlen und können in dieser Formel zur Anwendung verstäubt werden.

**Beispiel 3: Granulat**

Zur Herstellung eines 5 %-igen Granulates werden die folgenden Stoffe verwendet:

5 Teile Wirkstoff der Formel I
0,25 Teile epoxidiertes Pflanzenöl,
0,25 Teile Cetylpolyglykoläther,
3,50 Teile Polyäthylenglykol,
91 Teile Kaolin (Korngrösse 0,3 - 0,8 mm).

Die Aktivsubstanz oder die Mischung wird mit dem Pflanzenöl vermischt und mit 6 Teilen Aceton gelöst, hierauf wird Polyäthylenglykol und Cetylpolyglykoläther zugesetzt. Die so erhaltenene Lösung wird auf Kaolin aufgesprüht, und anschliessend wird das Aceton im Vakuum verdampft. Ein derartiges Mikrogranulat lässt sich vorteilhaft in Saatfurchen einarbeiten.

**Beispiel 4: Spritzpulver**

Zur Herstellung eines a) 70 %-igen, b) 40 %-igen, c) und d) 25 %-igen, e) 10 %-igen Spritzpulvers werden folgenden Bestandteile verwendet:

a) 70 Teile Wirkstoff der Formel I
5 Teile Natriumdibutylnaphthylsulfonat,
3 Teile Naphthalinsulfonsäure-Phenolsulfonsäuren-Formaldehyd-Kondensat 3 : 2 : 1,
10 Teile Kaolin,
12 Teile Champagne-Kreide,

b) 40 Teile Wirkstoff
5 Teile Ligninsulfonsäure-Natriumsalz,
1 Teil Dibutylnaphthalinsulfonsäure-Natriumsalz,
54 Teile Kieselsäure,

c) 25 Teile Wirkstoff
4,5 Teile Calcium-Ligninsulfonat,
1,9 Teile Champagne-Kreide/Hydroxyäthylcellulose-Gemisch (1 : 1),
1,5 Teile Natriumdibutylnaphthalinsulfonat,
19,5 Teile Kieselsäure,
19,5 Teile Champagne-Kreide,
28,1 Teile Kaolin,

d) 25 Teile Wirkstoff
2,5 Teile Isooctylphenoxypolyoxyäthylenäthanol,
1,7 Teile Champagne-Kreide/Hydroxyäthylcellulose-Gemisch (1 : 1),
8,3 Teile Natriumaluminiumsilikat,
16,5 Teile Kieselgur,
46 Teile Kaolin,

e) 10 Teile Wirkstoff
3 Teile Gemisch der Natriumsalze von gesättigten Fettalkoholsulfaten,
5 Teile Naphthalinsulfonsäure/Formaldehyd-Kondensat,
82 Teile Kaolin

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation (zwecks Wuchsverzögerung oder für Fungizideinsatz) verwenden lassen.

**Beispiel 5: Emulgierbare Konzentrate**

Zur Herstellung eines 25 %-igen emulgierbaren Konzentrates werden folgende Stoffe verwendet:

25 Teile Wirkstoff,
10 Teile eines Alkylarylsulfonat/Fettalkoholpolyglykoläther-Gemisches,
5 Teile Dimethylformamid,
57,5 Teile Xylol,
2,5 Teile epoxidiertes Pflanzenöl

**Beispiel 6: Paste**

Zur Herstellung einer 45 %-igen Paste werden folgende Stoffe verwendet:

a) 45 Teile Wirkstoff
5 Teile Natriumaluminiumsilikat
14 Teile Cetylpolyäthylenglykoläther mit 8 Mol Äthylenoxid,
1 Teil Oleylpolyäthylenglykoläther mit 5 Mol Äthylenoxid,
2 Teile Spindelöl,
23 Teile Wasser,
10 Teile Polyäthylenglykol,

b) 45 Teile des obigen Wirkstoffes oder der Mischung,
5 Teile Äthylenglykol,
3 Teile Octylphenoxypolyäthylenglykol mit 9 - 10 Mol Äthylenoxid pro Mol Octylphenol,
3 Teile von einem Gemisch aromatischer Sulfonsäuren, kondensiert mit Formaldehyd als Ammoniumsalz,
1 Teil Siliconöl in Form einer 75 %-igen Emulsion,
0,1 Teile einer Mischung von 1-(3-Chlorallyl)-3,5,7-triazoazonium-adamantanchlorid mit Natriumcarbonat, Chloridwert min. 11,5 %,
0,2 Teile eines biopolymeren Verdickers mit max. 100 Keimen pro Gramm,
42,7 Teile Wasser

Die Aktivsubstanz wird mit den Zuschlagstoffen in dazu geeigneten Geräten innig vermischt und vermahlen. Man erhält cine Paste, aus der sich durch Verdünnen mit Wasser Suspensionen jeder gewünschten Konzentration herstellen lassen.

**Biologische Beispiele:**

Die herbizide und den Pflanzenwuchs regulierende Wirkung wurde in den folgenden Versuchen ermittelt.

**Beispiel 7: Herbizide Wirkung bei Applikation des Wirkstoffes vor dem Auflaufen der Pflanzen (preemergent)**

Im Gewächshaus werden Pflanzensamen in Blumentöpfe mit ca. 11 cm Durchmesser gegeben, so dass sich pro Topf 12 - 25 Pflänzchen entwickeln können. Unmittelbar nach der Aussaat wird die Erdoberfläche mit einer wässrigen Emulsion der Prüfsubstanz behandelt, welche so verdünnt ist und gespritzt wird als ob eine Konzentration 4, 2, 1, 1/2 und 1/4 kg Substanz pro Hektar im Feld gespritzt würde.

Die Töpfe werden dann im Gewächshaus unter optimalen Bedingungen für den Pflanzenwuchs, d. h. bei regelmässigen Bewässern bei 22 - 25° C und 50 - 70 % relativer Luftfeuchtigkeit gehalten und der Versuch wird nach 3 Wochen ausgewertet und die Resultate nach folgender Notenskala bonitiert:

| | |
|---|---|
| 1 | Pflanzen nicht gekeimt oder abgestorben |
| 2 - 3 | Pflanzen nicht sehr stark |
| 4 - 6 | mittlere Schäden |
| 7 - 8 | geringe Schäden |
| 9 | keine Wirkung, die Pflanzen gedeihen wie unbehandelte Kontrollpflanzen |

Die Resultate sind unten zusammengestellt:

**Verbindung No. 1**

| Aufwandmenge kg/ha | 4 | 2 | 1 | 1/2 | 1/4 |
|---|---|---|---|---|---|
| Pflanze | | | | | |
| Avena fatua | 1 | 1 | 1 | 7 | 9 |
| Alopecurus myosuroides | 2 | 2 | 2 | 2 | 3 |
| Echinochloa crus galli | 1 | 1 | 1 | 1 | 2 |
| Rottboellia exaltata | 1 | 1 | 1 | 2 | 2 |
| Cyperus esculentus | 2 | 2 | 9 | 9 | 9 |
| Abutilon sp | 1 | 1 | 2 | 2 | 3 |
| Xanthium sp | 1 | 1 | 1 | 2 | 2 |
| Chenopodium album | 1 | 1 | 2 | 2 | 2 |
| Ipomoea purpurea | 2 | 2 | 2 | 3 | 6 |
| Sinapis alba | 2 | 2 | 2 | 3 | 4 |
| Galium aparine | 2 | 2 | 2 | 3 | 3 |
| Viola tricolor | 2 | 2 | 2 | 2 | 2 |
| Beta vulgaris | 2 | 2 | 2 | 3 | 3 |

**Beispiel 8: Herbizide Wirkung bei Applikation der Wirkstoffe nach dem Auflaufen der Pflanzen (postemergent)**

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässerigen Wirkstoffemulsionen (erhalten aus dem 25 %-igen Emulsionskonzentrat in Dosierungen von 4 und 2 kg/ha gespritzt. Die behandelten Pflanzen werden dann bei optimalen Bedingungen von Licht, regelmässigen Begiessen, 22 - 25° C Temperatur und 50 - 70 %-iger relativer Luftfeuchtigkeit gehalten. Die Auswertung des Versuches erfolgt 15 Tage nach der Behandlung und der Zustand der Pflanzen wird nach der oben gegebenen Skala bewertet. Die Resultate sind wie folgt:

**Verbindung No. 1**

| Aufwandmenge kg/ha | 4 | 2 |
|---|---|---|
| Pflanze | | |
| Avena fatua | 5 | 6 |
| Alopecurus myosuroides | 7 | 7 |
| Echinochloa crus galli | 4 | 4 |
| Rottboellia exaltata | 6 | 7 |
| Cyperus esculentus | 4 | 5 |
| Abutilon sp | 3 | 6 |
| Xanthium sp | 5 | 5 |
| Chenopodium album | 3 | 4 |
| Ipomoea purpurea | 4 | 5 |
| Sinapis alba | 4 | 4 |
| Galium apanne | 5 | 6 |
| Viola tricolor | 7 | 7 |
| Beta vulgaris | 3 | 3 |

**Beispiel 9: Selektive herbizide Wirkung auf Reis im Nachauflaufverfahren**

Reispflänzchen, welche 25 Tage alt sind, wurden im Gewächshaus in grosse rechteckige Eternitschalen verpflanzt. Zwischen die Reihen der Reispflanzen wurden dann Samen der in Reiskulturen vorkommenden Unkräuter Echinochloa, Scirpus, Monocharia und Sagittaria gesät. Die Schalen wurden gut bewässert und bei einer Temperatur von ca. 25°C und hoher Luftfeuchtigkeit gehalten. Nach 12 Tagen, wenn die Unkräuter aufgeluafen sind und das 2 - 3 Blattstadium erreicht haben, wurde die Erde in der Schale mit einer 2,5 cm hohen Schicht Wasser bedeckt. Der Wirkstoff wurde dann als Emulsionskonzentrat mittels einer Pipette zwischen die Pflanzenreihen appliziert, wobei man das Emulsionskonzentrat so verdünnte und auftrug, dass es einer Applikationsmenge im Feld 2, 1 1/2, 1/4 und 1/8 kg Wirkstoff pro Hektar entsprach. Der Versuch wurde nach 4 Wochen ausgewertet und der Zustand der Pflanzen nach der oben gegebenen Skala beurteilt. Die Resultate sind unten zusammengefasst.

**Verbindung No. 1**

| Aufwandmenge kg/ha | 2 | 1 | 1/2 | 1/4 | 1/8 |
|---|---|---|---|---|---|
| Pflanze | | | | | |
| Reis | 3 | 4 | 4 | 5 | 7 |
| Echinochloa crus galli | 1 | 1 | 1 | 1 | 1 |
| Scirpus sp. | 1 | 1 | 1 | 1 | 1 |
| Monocharia vaginalis | 1 | 1 | 1 | 1 | 1 |
| Sagittaria pygmea | 2 | 3 | 3 | 4 | 4 |

**Beispiel 10: Wuchshemmung bei Getreide**

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0.3 - 2.5 kg Aktivsubstanz pro Hektar. 10 und 21 Tage nach Applikation wurde das Wachstum des Getreides beurteilt. Hierbei konnte festgestellt werden, dass Getreidepflanzen, die mit Wirkstoffen der Formel I behandelt worden waren, im Vergleich zu unbehandelten Kontrollpflanzen eine starke Wuchsreduktion aufweisen. Als besonders wirksam erwies sich die Verbindung aus dem Beispiel 1. So reduzierte sie die Zuwachsrate auf 5 - 25 %.

**Beispiel 11: Wuchshemmung bei Gräsern**

In Kunststoffschalen mit Erde-Torf-Sand-Gemisch (6 : 3 : 1) wurden die Gräser Lolium perenne, Poa pratensis, Festuca orina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge betrug umgerechnet 0.3 - 2.5 kg Aktivsubstanz pro Hektar. 10 und 21 Tage nach Applikation wurde das Wachstum der Gräser beurteilt, dabei zeigte es sich, dass die erfindungsgemässen Wirkstoffe aus den Tabellen 1 bis 3 eine merkliche Wuchshemmung bewirkten. Besonders deutliche Wuchshemmung wurde mit der Verbindung aus dem Beispiel 1 bewirkt, so reduzierte sie das Weiterwachsen fast vollständig (Zuwachsrate ca. 10 %).

**Beispiel 12: Ertragssteigerung an Sojabohnen**

In Kunststoffbehältern mit einem Erde-Torf-Sand-Gemisch im Verhältnis 6 : 3 : 1 wurden Sojabohnen der Sorte "Hark" angesät und in eine Klimakammer gegeben. Durch optimale Temperaturwahl, Beleuchtung, Düngerzugabe und Bewässerung entwickelten sich die Pflanzen nach ca. 5 Wochen in dem 5 - 6 Trifola-Blattstadium. Zu diesem Zeitpunkt wurden die Pflanzen mit der wässrigen Brühe eines Wirkstoffes der Formel I bis zur guten Benetzung besprüht. Die Wirkstoffkonzentration betrug bis zu 500 ppm Aktivsubstanz. Die Auswertung erfolgte ca. 5 Wochen nach der Applikation des Wirkstoffs. Im Vergleich zu unbehandelten Kontrollpflanzen bewirkten die erfindungsgemässen Wirkstoffe der Formel I eine merkliche Erhöhung der Anzahl und des Gewichts der geernteten Schoten. Als besonders wirksam erwies sich die Verbindung aus dem Beispiel 1. Sie bewirkten je nach Versuch eine Ertragssteigerung von 5 bis 12 %.

**Beispiel 13: Wuchshemmung bei Bodenbedecker-Pflanzen (Cover-Crops)**

In Kunststoffschalen mit Erde-Torf-Sand-Gemisch (1 : 1 : 1) werden Testpflanzen der Sorte Centrosema plumieri und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70 % statt. Die Testpflanzen werden auf eine Höhe von ca. 15 cm zurückgeschnitten und 7 Tage nach dem Zurückschneiden mit einer Spritzbrühe des Wirkstoffes (umgerechnet zw. 0.3 - 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass die Verbindung aus dem Beispiel 1 eine deutliche Wuchshemmung der Bodenbedecker-Pflanzen auslöst. Sie bewirkt eine starke Wuchshemmung und reduzierten die Zuwachsrate auf ca. 10 %.

**Beispiel 14: Seneszenzhemmung bei Getreidepflanzen**

Im Gewächshaus wird Sommerweizen der Sorte "Svenno" in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23°C, 70 % relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass Verbindungen aus dem Beispiel 1 eine deutliche Inhibierung der Seneszenz der Testpflanzen hervorruft. Sie hemmten das Vergilben der Blätter im Versuchszeitraum um mehr als 80 %.

## Patentansprüche

1. 1-Di- oder Triazolyl-2,3-diphenyl-propan-2,3-diole und Derivate der Formel I

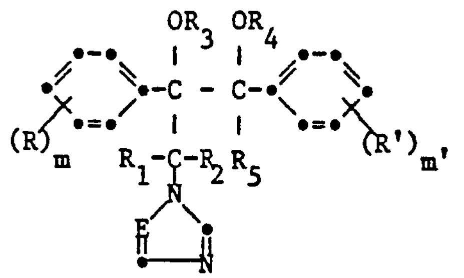

(I),

worin
E ein Stickstoffatom oder die Methingruppe -CH=,
m und m' unabhängig voneinander 0, 1, 2 oder 3,
R und R' unabhängig voneinander Halogen, $C_1$-$C_4$ Alkyl, $C_1$-$C_4$ Halogenalkyl, $C_1$-$C_4$ Alkoxy, $C_1$-$C_4$ Halogenalkoxy, Nitro, Cyano, $C_1$-$C_4$ Alkylthio, Phenyl oder Phenoxy, welches unsubstituiert oder gegebenenfalls durch $(R)_m$ substituiert ist, ferner $C_1$-$C_4$ Alkoxycarbonyl, $C_1$-$C_4$ Halogenalkylthio, Amino, Mono- oder Di-($C_1$-$C_4$ alkyl)amino,
$R_1$ und $R_2$ unabhängig voneinander je Wasserstoff oder $C_1$-$C_4$ Alkyl,
$R_3$ und $R_4$ je einzeln Wasserstoff $C_1$-$C_4$ Alkylcarbonyl,
$R_3$ und $R_4$ zusammen auch eine Methylenbrücke $-CR_6R_7-$,
$R_5$ Wasserstoff oder $C_1$-$C_4$ Alkyl,
$R_6$ und $R_7$ je einzeln Wasserstoff oder $C_1$-$C_4$ Alkyl,
$R_6$ und $R_7$ zusammen mit dem Kohlenstoffatom, an das sie gebunden sind einen 3 - 6 gliedrigen gesättigten oder ungesättigten Kohlenwasserstoffring.

2. 3-Di- oder Triazolyl-1,2-diphenyl-propan-1,2-diole gemäss Anspruch 1 der Formel Ia

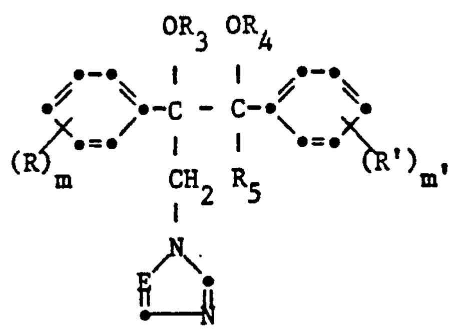

(Ia),

worin E, m, m', R, R', $R_3$, $R_4$ und $R_5$ die im Anspruch 1 gegebene Bedeutung haben.

3. 1-(1,2,4-Triazol-1H-1-yl)-2,3-diphenylpropan-2,3-diolderivate gemäss Anspruch 1 der Formel Ib,

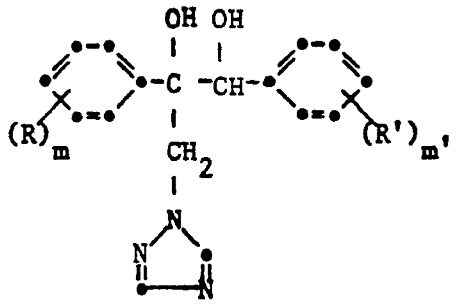

(Ib),

worin m und m' unabhängig voneinander 0, 1 oder 2 und R und R' unabhängig voneinander Halogen, $C_1$-$C_4$-Alkyl, $C_1$-$C_4$ Alkoxy oder $C_1$-$C_4$-Halogenalkyl bedeuten.

4. 2-(2,4-Dichlorphenyl)-3-phenyl-1-(1,2,4-triazol-1H-1-yl)-propan-2,3-diol gemäss Anspruch 1.

5. 2-(4-Chlorphenyl)-3-phenyl-1-(1,2,4-triazol-1H-1-yl)-propan-2,3-diol gemäss Anspruch 1.

6. 2-(4-Chlorphenyl)-3-(2-chlorphenyl)-1-(1,2,4-triazol-1H-1-yl)-propan-2,3-diol gemäss Anspruch 1.

7. 2-(4-Chlorphenyl)-3-para-tolyl-1-(1,2,4-triazol-1H-1-yl)-propan-2,3-diol gemäss Anspruch 1.

8. 2-(4-Chlorphenyl)-3-(4-methoxyphenyl)-1-(1,2,4-triazol-1H-1-yl)-propan-2,3-diol gemäss Anspruch 1.

9. 2-(4-Chlorphenyl)-3-(4-fluorphenyl)-1-(1,2,4-triazol-1H-1-yl)-propan-2,3-diol gemäss Anspruch 1.

10. 1-(Imidazol-1H-1-yl)-2,3-diphenyl-propan-2,3-diole und Derivate gemäss Anspruch 1 der Formel Ic

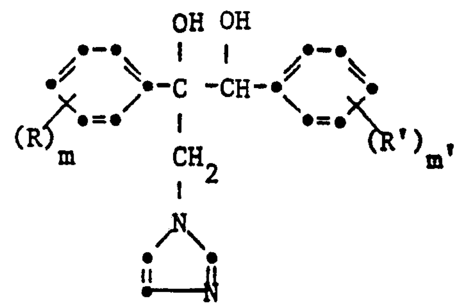

(Ic),

worin m und m' unabhängig voneinander 0, 1 oder 2 und R und R' unabhängig voneinander Halogen, $C_1$-$C_4$-Alkyl, $C_1$-$C_4$-Alkoxy oder $C_1$-$C_4$-Halogenalkyl bedeuten.

11. 1-(1,3,4-Triazol-1H-1-yl)-2,3-diphenyl-2,3-diole und Derivate gemäss Anspruch 1 der Formel Id

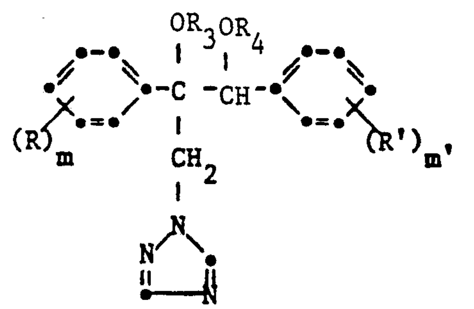

(Id),

worin m und m' unabhängig voneinander 0, 1 oder 2, R und R' unabhängig voneinander Halogen, $C_1$-$C_4$ Alkyl, $C_1$-$C_4$ Alkoxy oder $C_1$-$C_4$ Halogenalkyl, $R_3$ und $R_4$ je einzeln $C_1$-$C_4$ Alkyl, $C_1$-$C_4$ Alkylcarbonyl oder eines auch Wasserstoff oder zusammen eine 3 - 6 gliedrige Alkylenbrücke, die ein- oder zweifach durch $C_1$-$C_4$ Alkyl substituiert sein kann.

12. 2,2-Dimethyl-4-phenyl-5-(2,4-dichlorphenyl)-5-(1,2,4-triazol-1H-1-ylmethyl)-1,3-dioxolan gemäss Anspruch 1 der Formel

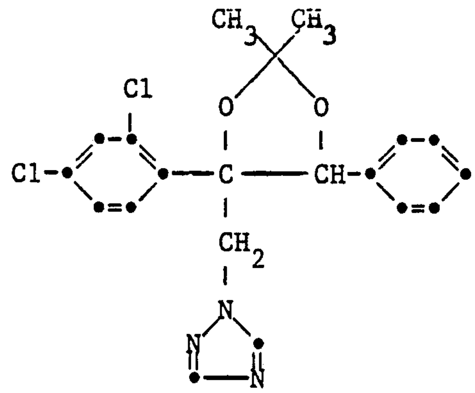

13. 1-(1,3,4-Triazol-1H-1-yl)-2-(2,4-dichlorphenyl-2-methoxy-3-phenyl-propan-3-ol gemäss Anspruch 1.

14. Verfahren zur Herstellung der 3-Di- oder Triazolyl-1,2-diphenyl-propan-1,2-diole der Formel I, indem man Verbindungen der Formel III

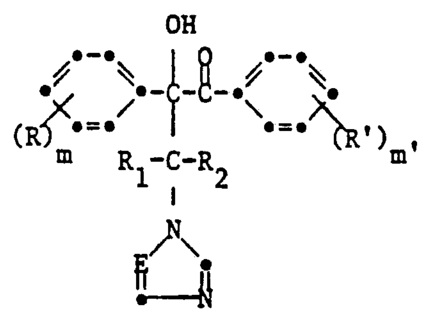 (III),

worin E, m, m', R, R', $R_1$ und $R_2$ die im Anspruch 1 gegebene Bedeutung haben, in einem inerten organischen Lösungsmittel, mit einem komplexen Metallhydrid, mit Wasserstoff und einem Katalysator, mit einer Lithium (alkyl)verbindung $LiR_5$ oder einem Alkyl-Magnesiumhalogenid $R_5Mg$ Hal, und das erhaltene Diol der Formel IV reduziert

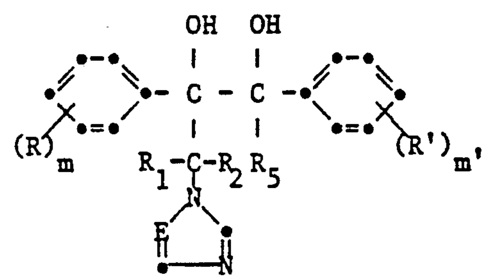 (IV),

worin E, m, m', R, R', $R_1$, $R_2$ und $R_5$ die im Anspruch 1 gegebene Bedeutung haben, gegebenenfalls anschliessend in einem inerten organischen Lösungsmittel, in Gegenwart einer äquimolaren Base als Säureakzeptor in einer oder zwei Stufen mit je einem Mol $R_3$-X und $R_4$-X, welche identisch sein können, alkyliert, oder die Verbindung der Formel IV in Gegenwart einer starken Säure mit einem Keton der Formel V kondensiert,

$$\begin{array}{l} O = C - R_7 \\ \quad\;\; | \\ \quad\;\; R_6 \end{array} \qquad (V)$$

in welchen Formeln $R_3$, $R_4$, $R_6$ und $R_7$ die im Anspruch 1 gegebene Bedeutung haben und das entsprechende Produkt aus dem Reaktionsgemisch isoliert.

15. Herbizides und den Pflanzenwuchs regulierendes Mittel, dadurch gekennzeichnet, dass es als Wirkstoff ein 1-Di- oder Triazol-2,3-diphenyl-propan-2,3-diol der Formel I, Anspruch 1 enthält zusammen mit inerten Trägerstoffen.

16. Verfahren zur Herstellung eines herbiziden oder den Pflanzenwuchs regulierenden Mittels, dadurch gekennzeichnet, dass man ein 1-Di- oder Triazolyl-2,3-diphenyl-propan-2,3-diol der Formel I gemäss Anspruch 1 mit geeigneten festen oder flüssigen Zusatzstoffen und Tensiden innig vermischt.

17. Verfahren zur Bekämpfung von Unkräutern, dadurch gekennzeichnet, dass man eine wirkungsvolle Menge einer Verbindung der Formel I gemäss Anspruch 1 auf die Unkräuter oder deren Standort appliziert.

18. Verfahren zur Regulierung des Pflanzenwuchses, dadurch gekennzeichnet, dass man eine wirkungsvolle Menge einer Verbindung der Formel I gemäss Anspruch 1 auf die Pflanzen oder Pflanzenteile, deren Wachstum man zu beeinflussen wünscht, appliziert.

19. Die Verwendung der Verbindungen der Formel I, Anspruch 1 als Herbizide oder als Mittel zur Regulierung des Pflanzenwuchses.

20. Die Verwendung der Verbindungen der Formel I, Anspruch 1 zur Wuchshemmung im Sinne einer Erhöhung der Knickfestigkeit und Halmverkürzung bei Getreidesorten.

21. Die Verwendung der Verbindungen der Formel I, Anspruch 1 zur Wuchshemmung bei Bodenbedeckerpflanzen.

22. Die Verwendung der Verbindungen der Formel I, Anspruch 1 zur Wuchshemmung bei Bodenbedeckerpflanzen.

23. Die Verwendung der Verbindungen der Formel I, Anspruch 1 zur Wuchsregulierung bei Leguminosen im Sinne einer Ertragssteigerung.

24. Verfahren zum selektiven Bekämpfen von Unkräutern in Nutzpflanzenkulturen, dadurch gekennzeichnet, dass man die Kulturen oder deren Anbaufläche mit einer wirksamen Menge einer 1-Di- oder Triazolyl-2,3-diphenyl-propan-2,3-dioles oder -Derivates der Formel I gemäss Anspruch 1 behandelt.

25. Verfahren zum selektiven Bekämpfen von Unkräutern in Kulturen von Getreide, Reis, Hirse und Mais, dadurch gekennzeichnet, dass man die Kulturen oder deren Anbaufläche mit einer wirksamen Menge eines 1-Di- oder Triazilyl-2,3-diphenyl-propan-2,3-diols oder Derivates der Formel I gemäss Anspruch 1 behandelt.

**Claims**

1. 1-diazolyl- or 1-triazolyl-2,3-diphenylpropane-2,3-diols and derivatives of the formula I

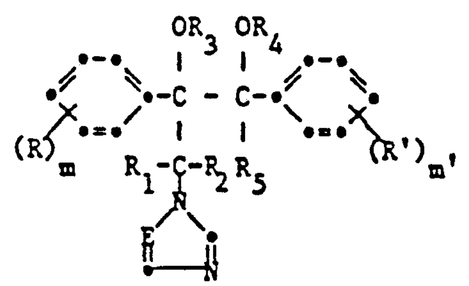

(I)

wherein

E is a nitrogen atom or the methine group -CH=,

m and m' are each independently of the other 0, 1, 2 or 3,

R and R' are each independently of the other halogen, $C_1$-$C_4$alkyl, $C_1$-$C_4$haloalkyl, $C_1$-$C_4$alkoxy, $C_1$-$C_4$haloalkoxy, nitro, cyano, $C_1$-$C_4$alkylthio, phenyl or phenoxy, each unsubstituted or substituted by $(R)_m$, or are $C_1$-$C_4$alkoxycarbonyl, $C_1$-$C_4$haloalkylthio, amino, mono- or di-($C_1$-$C_4$ alkyl)amino,

$R_1$ and $R_2$ are each independently of the other hydrogen or $C_1$-$C_4$alkyl,

$R_3$ and $R_4$ are each independently of the other hydrogen, $C_1$-$C_4$alkyl or $C_1$-$C_4$alkylcarbonyl, or $R_3$ and $R_4$ together are a methylene bridge $-CR_6R_7-$,

$R_5$ is hydrogen or $C_1$-$C_4$alkyl, and

$R_6$ and $R_7$ are each independently of the other hydrogen or $C_1$-$C_4$lkyl or, together with the carbon atom to which they are attached, are a 3- to 6-membered saturated or unsaturated hydrocarbon ring.

2. 3-Diazolyl- or 3-triazolyl-1,2-diphenylpropane-1,2-diols according to claim 1 of the formula Ia

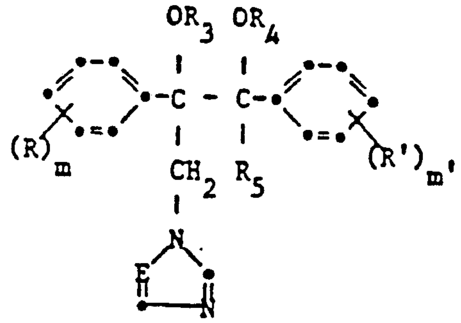

(Ia),

wherein E, m, m', R, R', $R_3$, $R_4$ and $R_5$ are as defined in claim 1.

3. 1-(1,2,4-Triazol-1H-1-yl)-2,3-diphenylpropane-2,3-diol derivatives according to claim 1 of the formula Ib

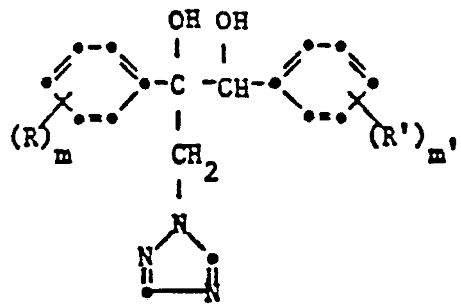

(Ib),

wherein each of m and m' independently of the other is 0, 1 or 2, and each of R and R' independently of the other is halogen, $C_1$-$C_4$alkyl, $C_1$-$C_4$alkoxy or $C_1$-$C_4$haloalkyl.

4. 2-(2,4-Dichlorophenyl)-3-phenyl-1-(1,2,4-triazol-1H-1-yl)-propane-2,3-diol according to claim 1.

5. 2-(4-Chlorophenyl)-3-phenyl-1-(1,2,4-triazol-1H-1-yl)-propane-2,3-diol according to claim 1.

6. 2-(4-Chlorophenyl)-3-(2-chlorophenyl)-1-(1,2,4-triazol-1H-1-yl)-propane-2,3-diol according to claim 1.

7. 2-(4-Chlorophenyl)-3-para-tolyl-1-(1,2,4-triazol-1H-1-yl)-propane-2,3-diol according to claim 1.

8. 2-(4-Chlorophenyl)-3-(4-methoxyphenyl)-1-(1,2,4-triazol-1H-1-yl)-propane-2,3-diol according to claim 1.

9. 2-(4-Chlorophenyl)-3-(4-fluorophenyl)-1-(1,2,4-triazol-1H-1-yl)-propane-2,3-diol according to claim 1.

10. 1-(Imidazol-1H-1-yl)-2,3-diphenylpropane-2,3-diols and derivatives according to claim 1 of the formula Ic

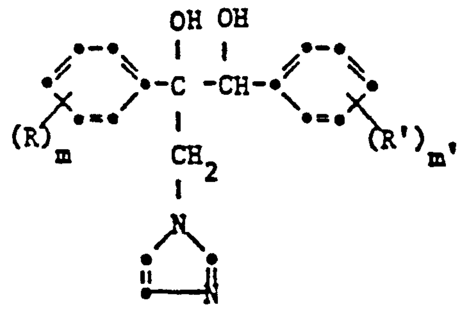

(Ic),

wherein each of m and m' independently of the other is 0, 1 or 2 and each of R and R' independently of the other is halogen, $C_1$-$C_4$alkyl, $C_1$-$C_4$alkoxy or $C_1$-$C_4$haloalkyl.

11. 1-(1,3,4-Triazol-1H-1-yl)-2,3-diphenyl-2,3-diols and derivatives according to claim 1 of the formula Id

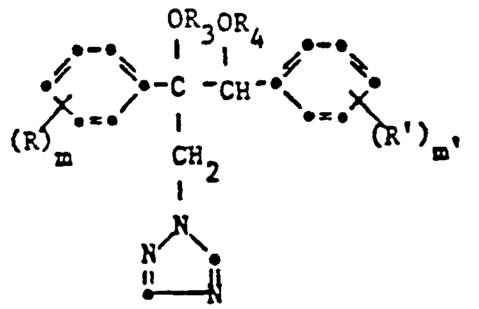

(Id),

wherein each of m and m' independently of the other is 0, 1 or 2, each of R and R' independently of the other is halogen, $C_1$-$C_4$alkyl, $C_1$-$C_4$alkoxy or $C_1$-$C_4$haloalkyl, and each of $R_3$ and $R_4$ independently of the other is $C_1$-$C_4$alkyl, $C_1$-$C_4$alkylcarbonyl, or one of $R_3$ and $R_4$ is also hydrogen, or the two together form a 3- to 6-membered alkylene bridge which may be substituted by one or two $C_1$-$C_4$alkyl groups.

12. 2,2-Dimethyl-4-phenyl-5-(2,4-dichlorophenyl)-5-(1,2,4-triazol-1H-1-ylmethyl)-1,3-dioxolane according to claim 1 of the formula

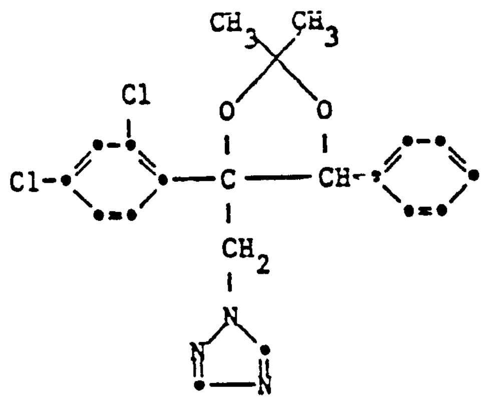

13. 1-(1,3,4-Triazol-1H-1-yl)-2-(2,4-dichlorophenyl-2-methoxy)-3-phenylpropan-3-ol according to claim 1.

14. A process for the preparation of the 3-diazolyl- or 3-triazolyl-1,2-diphenylpropane-1,2-diols of the formula I, which comprises reducing compounds of the formula III

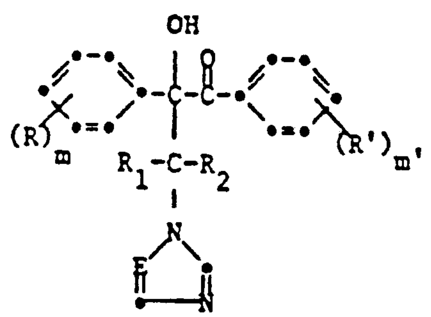

(III),

wherein E, m, m', R, R', $R_1$ and $R_2$ are as defined in claim 1, in an inert organic solvent, with a complex metal hydride, with hydrogen and a catalyst, with a lithium(alkyl) compound $LiR_5$ or with an alkylmagnesium halide $R_5MgHal$, and optionally then alkylating the resulting diol of the formula IV

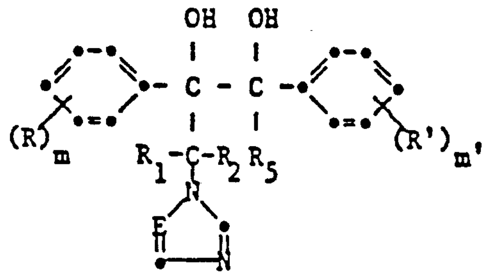

(IV),

wherein E, m, m', R, R', $R_1$, $R_2$ and $R_5$ are as defined in claim 1, in one or two steps with one mole of each of $R_3$-X and $R_4$-X, which can be identical, in an inert organic solvent in the presence of the equimolar amount of a base as acid acceptor, or condensing the compound of the formula IV, in the presence of a strong acid, with a ketone of the formula V

$$O = C - R_7 \quad (\text{with } R_6 \text{ on C}) \qquad (V)$$

in which formulae $R_3$, $R_4$, $R_6$ and $R_7$ are as defined in claim 1, and isolating the corresponding product from the reaction mixture.

15. A herbicidal and plant growth-regulating composition which contains as active ingredient a 1-diazole- or 1-triazole-2,3-diphenylpropane-2,3-diol of the formula I, claim 1, together with inert carriers.

16. A process for the preparation of a herbicidal or plant growth-regulating composition, which comprises intimately mixing a 1-diazolyl- or 1-triazolyl-2,3-diphenylpropane-2,3-diol of the formula I according to claim 1, with suitable solid or liquid adjuvants and surfactants.

17. A method of controlling weeds, which comprises applying to the weeds or to the locus thereof an effective amount of a compound of formula 1 according to claim 1.

18. A method of regulating plant growth, which comprises applying to the plants or parts of plants whose growth it is desired to influence an effective amount of a compound of formula 1 according to claim 1.

19. The use of the compounds of formula 1, claim 1, as herbicides or as compositions for regulating plant growth.

20. The use of the compounds of formula I, claim 1, to inhibit growth in order to increase resistance to lodging and to shorten stalks in cereal varieties.

21. The use of the compounds of formula I, claim 1, to inhibit growth in cover crops.

22. The use of the compounds of formula I, claim 1, to inhibit growth in cover crops.

23. The use of the compounds of formula I, claim 1, to regulate growth in legumes for the purpose of increasing yield.

24. A method of selectively controlling weeds in crops of useful plants, which comprises treating the crops or the area under cultivation with an effective amount of a 1-diazolyl- or 1-triazolyl-2,3-diphenylpropane-2,3-diol or derivative of the formula I according to claim 1.

25. A method of selectively controlling weeds in crops of cereals, rice, sorghum and maize, which comprises treating the crops or the area under cultivation with an effective amount of a 1-diazolyl- or 1-triazolyl-2,3-diphenylpropane-2,3-diol or derivative of the formula 1 according to claim 1.

## Revendications

1. 1-di- ou triazolyl-2,3-diphényl-propane-2,3-diols et dérivés de formule I

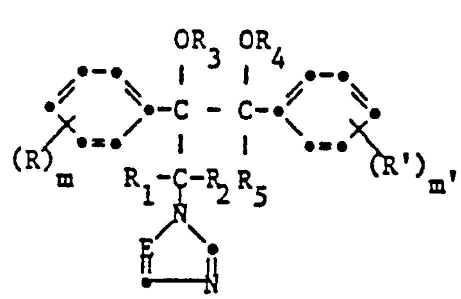

(I),

dans laquelle

E représente un atome d'azote ou le groupe méthine -CH=,

m et m' sont egaux, indépendamment l'un de l'autre, à 0, 1, 2 ou 3,

R et R' représentent chacun, indépendamment l'un de l'autre, un halogène, un groupe alkyle en $C_1$-$C_4$, halogénoalkyle en $C_1$-$C_4$, alcoxy en $C_1$-$C_4$, halogénoalcoxy en $C_1$-$C_4$, nitro, cyano, alkylthio en $C_1$-$C_4$, phényle ou phénoxy non substitué ou éventuellement substitué par $(R)_m$, ou encore un groupe (alcoxy en $C_1$-$C_4$)-carbonyle, halogénoalkylthio en $C_1$-$C_4$, amino, mono- ou di-(alkyle en $C_1$-$C_4$)-amino,

$R_1$ et $R_2$ représentent chacun, indépendamment l'un de l'autre, l'hydrogène ou un groupe alkyle en $C_1$-$C_4$,

$R_3$ et $R_4$ représentent chacun, indépendamment l'un de l'autre, l'hydrogène, un groupe alkyle en $C_1$-$C_4$, (alkyle en $C_1$-$C_4$)-carbonyle ou forment ensemble un pont méthylène $-CR_6R_7-$,

$R_5$ représente l'hydrogène ou un groupe alkyle en $C_1$-$C_4$,

$R_6$ et $R_7$ représentent chacun, indépendamment l'un de l'autre, l'hydrogène ou un groupe alkyle en C1-C4, $R_6$ et $R_7$ forment ensemble avec l'atome de carbone auquel ils sont reliés, un cycle hydrocarboné saturé ou insaturé de 3 a 6 chaînons.

2. 3-di- ou triazolyl-1,2-diphényl-propane-1,2-diols selon la revendication 1, répondant à la formule Ia

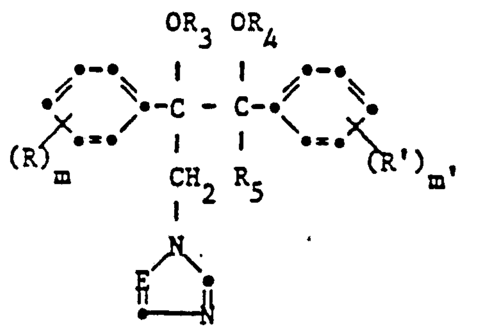

(Ia),

dans laquelle E, m, m', R, R', $R_3$, $R_4$ et $R_5$ ont les significations indiquées dans la revendication 1.

3. Dérivés de 1-(1,2,4-triazole-1H-1-yl)-2,3-diphénylpropane-2,3-diols selon la revendication 1, répondant à la formule Ib,

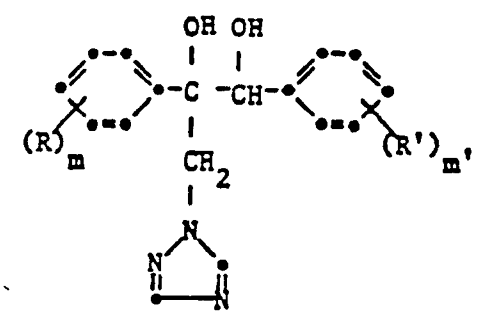

(Ib),

dans laquelle m et m' sont chacun égaux, indépendamment l'un de l'autre, à 0, 1 ou 2 et R et R' représentent chacun, indépendamment l'un de l'autre, un halogène, un groupe alkyle en $C_1$-$C_4$, alcoxy en $C_1$-$C_4$ ou halogénoalkyle en $C_1$-$C_4$.

4. Le 2-(2,4-dichlorophényl)-3-phényl-1-(1,2,4-triazole-1H-1-yl)-propane-2,3-diol selon la revendication 1.

5. Le 2-(4-chlorophényl)-3-phényl-1-(1,2,4-triazole-1H-1-yl)-propane-2,3-diol selon la revendication 1.

6. Le 2-(4-chlorophényl)-3-(2-chlorophényl)-1-(1,2,4-triazole-1H-1-yl)-propane-2,3-diol selon la revendication 1.

7. Le 2-(4-chlorophényl)-3-p-tolyl-1-(1,2,4-triazole-1H-1-yl)-propane-2,3-diol selon la revendication 1.

8. Le 2-(4-chlorophényl)-3-(4-méthoxyphényl)-1-(1,2,4-triazole-1H-1-yl)-propane-2,3-diol selon la revendication 1.

9. Le 2-(4-chlorophényl)-3-(4-fluorophényl)-1-(1,2,4-triazole-1H-1-yl)-propane-2,3-diol selon la revendication 1.

10. Les 1-(imidazole-1H-1-yl)-2,3-diphényl-propane-2,3-diols et dérivés selon la revenication 1, répondant à la formule Ic

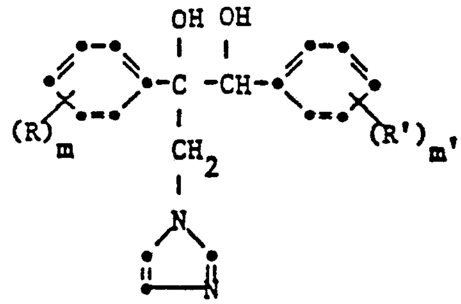

(Ic),

dans laquelle m et m' sont chacun égaux, indépendamment l'un de l'autre, à 0, 1 ou 2 et R et R' représentent chacun, indépendamment l'un de l'autre, un halogène, un groupe alkyle en $C_1$-$C_4$, alkoxy en $C_1$-$C_4$ ou halogénoalkyle en $C_1$-$C_4$.

11. Les 1-(1,3,4-triazole-1H-1-yl)-2,3-diphényl-2,3-diols et dérivés selon la revendication 1, répondant à la formule Id

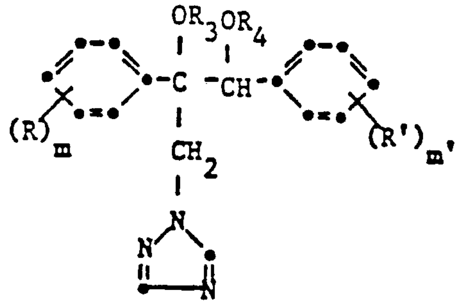

(Id),

dans laquelle m et m' sont chacun égaux, indépendamment l'un de l'autre, à 0, 1 ou 2, R et R' représentent chacun, indépendamment l'un de l'autre, un halogène, un groupe alkyle en $C_1$-$C_4$, alcoxy en $C_1$-$C_4$ ou halogénoalkyle en $C_1$-$C_4$, $R_3$ et $R_4$ représentent chacun, indépendamment l'un de l'autre, un groupe alkyle en $C_1$-$C_4$, (alkyle en $C_1$-$C_4$)-carbonyle, ou bien l'un de ces symboles peut également représenter l'hydrogène, ou bien $R_3$ et $R_4$ forment ensemble un pont alkylène de 3 à 6 chaînons qui peut être substitué par un ou deux groupes alkyle en $C_1$-$C_4$.

12. Le 2,2-diméthyl-4-phényl-5-(2,4-dichlorophényl)-5-(1,2,4-triazole-1H-1-ylméthyl)-1,3-dioxolanne selon la revendication 1, de formule

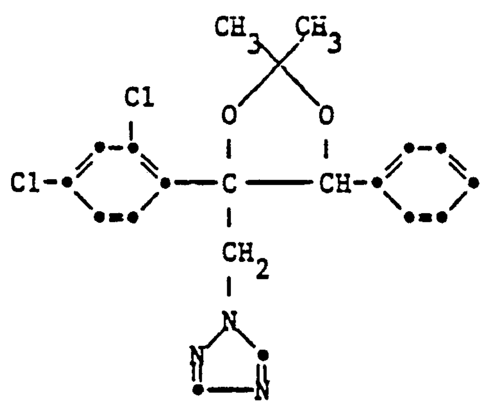

13. Le 1-(1,3,4-triazole-1H-1-yl)-2-(2,4-dichlorophényl)-2-méthoxy-3-phényl-propane-3-ol selon la revendication 1.

14. Procédé de préparation des 3-di- ou triazolyl-1,2-diphénylpropane-1,2-diols de formule I, qui consiste à réduire des composés de formule III

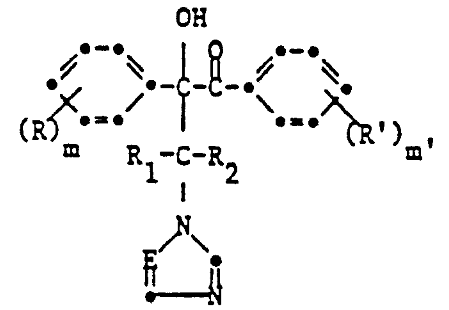

(III),

dans laquelle E, m, m', R, R', $R_1$ et $R_2$ ont les significations indiquées dans la revendication 1, dans un solvant organique inerte, à l'aide d'un hydrure métallique complexe, à l'aide de l'hydrogène et d'un catalyseur, à l'aide d'un composé d'alkyl-lithium $LiR_5$ ou d'un halogénure d'alkylmagnésium $R_5Mg$ Hal, ce qui donne le diol de formule IV

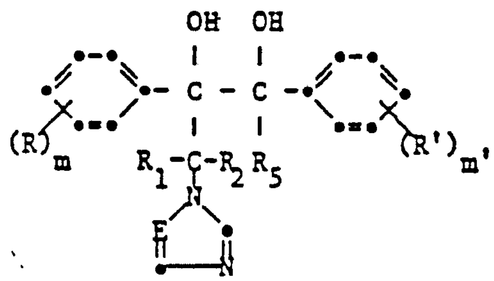

(IV),

dans laquelle E, m, m', R, R', $R_1$, $R_2$ et $R_3$ ont les significations indiquées dans la revendication 1, qu'on soumet ensuite le cas échéant à alkylation dans un solvant organique inerte, en présence d'une base équimoléculaire servant d'accepteur d'acide, en un ou deux stades opératoires, par 1 mole de $R_3$-X et 1 mole de $R_4$-X, ces deux réactifs pouvant être identiques, ou bien on condense le composé de formule IV en presence d'un acide fort avec une cétone de formule V

$$O = \underset{\displaystyle R_6}{\underset{|}{C}} - R_7 \qquad (V)$$

les symboles $R_3$, $R_4$, $R_6$ et $R_7$ des formules ci-dessus ayant les significations indiquées dans la revendication 1, après quoi on isole le produit obtenu du mélange de reaction.

15. Produit herbicide et régulateur de la croissance des végétaux, caractérisé en ce qu'il contient en tant que substance active, avec des véhicules inertes, un 1-di- ou triazole-2,3-diphényl-propane-2,3-diol de formule I, revendication 1.

16. Procédé de préparation d'un produit herbicide ou régulateur de la croissance des végétaux, caractérisé en ce que l'on mélange intimement un 1-di-ou triazolyl-2,3-diphényl-propane-2,3-diol de formule I selon la revendication 1 avec des additifs solides ou liquides et agents tensio-actifs appropriés.

17. Procédé pour combattre les végétaux adventices, caractérisé en ce que l'on applique sur ces végétaux ou leur habitat une quantité efficace d'un composé de formule I selon la revendication 1.

18. Procédé pour la régulation de la croissance des vegetaux, caractérisé en ce que l'on applique sur les végétaux ou des parties des végétaux dont on veut influencer la croissance une quantité efficace d'un composé de formule I selon la revendication 1.

19. L'utilisation des composés de formule I, revendication 1, en tant qu'herbicide ou produit régulateur de la croissance des végétaux.

20. L'utilisation des composés de formule I, revendication 1, pour l'inhibition de la croissance en vue d'une augmentation de la résistance au couchage et d'un raccourcissement de la tige chez les céréales.

21. L'utilisation des composés de formule I, revendication 1, pour l'inhibition de la croissance des végétaux de couverture de sol.

22. L'utilisation des composés de formule I, revendication 1, pour l'inhibition de la croissance des végétaux de couverture de sol.

23. L'utilisation des composés de formule I, revendication 1, pour la régulation de la croissance des légumineuses en vue d'une augmentation du rendement.

24. Procédé pour combattre sélectivement les végétaux adventices dans les cultures de végétaux utiles,

caractérisé en ce que l'on traite les plantes cultivées ou leurs aires de culture par une quantité efficace d'un 1-di- ou triazolyl-2,3-diphényl-propane-2,3-diol ou dérivé de formule I selon la revendication 1.

25. Procédé pour combattre sélectivement les végétaux adventices dans les cultures de céréales, de riz, de millet et de mais, caractérisé en ce que l'on traite les plantes cultivées ou leurs aires de culture par une quantité efficace d'un 1-di- ou triazolyl-2,3-diphényl-propane-2,3-diol ou dérivé de formule I selon la revendication 1.